# FASCICULE DE BREVET EUROPEEN

(11) **EP 0 575 239 B1**
(45) Date de publication et mention de la délivrance du brevet: **23.10.1996**
(21) Numéro de dépôt: 93401526.4
(22) Date de dépôt: 15.06.1993
(51) Int. Cl.: B05B 11/06, A61M 11/02

(54) **Dispositif à gaz comprimé pour projeter sous forme finement divisée une dose unique d'une substance fluide**
Druckgasvorrichtung zum einmaligen Versprühen einer begrenzten Menge eines flüssigen Materials in fein verteilten Teilchen
Compressed gas device for spraying a single dose of fluid material in a finely divided form

(30) Priorité: 16.06.1992 FR 9207250
(43) Date de publication de la demande: 22.12.1993
(73) Titulaire: ETABLISSEMENTS VALOIS Société Anonyme dite:, F-27110 Le Neubourg (FR)
(72) Inventeur: Petit, Ludovic, F-27370 Amfreville la Campagne (FR); Solignac, Philippe, Résidence Vallon, F-76000 Rouen (FR); Bruna, Pascal, F-76000 Rouen (FR)
(74) Mandataire: Pinguet, André

(56) Documents cités:
- FR-A- 2 256 084
- US-A- 2 223 611

## Description

La présente invention concerne un dispositif à gaz comprimé pour projeter sous forme finement divisée une dose unique d'une substance fluide.

Plus particulièrement, l'invention s'applique à de tels dispositifs destinés à pulvériser une dose unique d'un médicament sous forme solide pulvérulente ou sous forme liquide. De tels dispositifs sont généralement utilisés pour la pulvérisation nasale.

Le document CH-A-518 744 divulgue un dispositif à gaz comprimé pour projeter sous forme finement divisée une dose unique d'une substance fluide, comportant:
- un réservoir contenant ladite dose unique, ledit réservoir ayant un orifice d'entrée de gaz comprimé et un orifice de sortie,
- des moyens de compression de gaz à actionnement manuel, lesdits moyens de compression de gaz comportant une réserve de gaz qui communique avec l'orifice d'entrée du réservoir par l'intermédiaire d'un canal intermédiaire comportant un tronçon de section rétrécie, ledit tronçon de section rétrécie étant fermé de façon étanche par un bouchon qui est coincé dans ledit tronçon de section rétrécie, de façon que lorsque la pression du gaz contenu dans la réserve de gaz atteint un seuil prédéterminé, ledit bouchon soit expulsé dudit tronçon de section rétrécie vers le réservoir de substance, en libérant ledit canal intermédiaire,
le dispositif comportant des moyens pour limiter le déplacement du bouchon vers l'orifice de sortie du réservoir de substance en définissant ainsi une position de butée du bouchon.

Dans le document susmentionné, le réservoir de substance comporte une partie convergente, qui converge vers l'orifice de sortie, et qui constitue les moyens pour limiter le déplacement du bouchon vers l'orifice de sortie du réservoir. Le bouchon a la forme d'une bille sphérique, de sorte qu'il existe un risque non négligeable pour que la bille vienne obstruer l'orifice de sortie avant que la totalité de la substance contenue dans le réservoir ne soit expulsée.

La présente invention a pour but de supprimer cet inconvénient.

La présente invention a donc pour objet un dispositif à gaz comprimé tel que défini ci-dessus, caractérisé en ce que lesdits moyens pour limiter le déplacement du bouchon présentent des moyens pour garantir le passage du flux de gaz comprimé lorsque le bouchon est dans sa position de butée.

Selon une forme de réalisation, le réservoir de substance comporte une partie convergente, qui converge vers l'orifice de sortie, ladite partie convergente constitue lesdits moyens pour limiter le mouvement du bouchon, et ladite partie convergente comporte des reliefs intérieurs qui constituent lesdits moyens pour garantir le passage du flux de gaz comprimé.

Selon une autre forme de réalisation, ladite substance est un matériau solide pulvérulent, le réservoir de substance comporte une barrière fixe, qui maintient la dose de matériau pulvérulent dans une position éloignée de l'orifice d'entrée de gaz comprimé, ladite barrière fixe constitue lesdits moyens pour limiter le mouvement du bouchon, et lesdits moyens pour garantir le passage du flux de gaz comprimé comportent au moins une ouverture qui traverse ladite barrière fixe. Avantageusement, dans cette forme de réalisation, lorsque le bouchon entre en contact avec ladite barrière fixe, ledit bouchon possède une énergie cinétique suffisante pour que le choc du bouchon sur ladite barrière fixe entraîne un décollement de la dose de matériau pulvérulent par rapport à ladite barrière fixe. Ladite barrière fixe peut être un élément en forme de membrane, avantageusement dotée d'une certaine flexibilité. Ladite barrière fixe peut aussi comporter une tige centrale pleine dotée d'au moins une rainure latérale axiale constituant ladite au moins une ouverture de passage du flux de gaz comprimé.

Selon une autre forme de réalisation, le bouchon comporte un talon élargi qui coopère avec le tronçon de section rétrécie du canal intermédiaire pour limiter le mouvement du bouchon vers le réservoir de substance, et ledit talon comporte au moins une ouverture qui constitue lesdits moyens pour garantir le passage du flux de gaz comprimé.

Avantageusement, lorsque ladite substance est un matériau solide pulvérulent, au moment où lesdits moyens pour limiter le mouvement du bouchon arrêtent le déplacement dudit bouchon, ledit bouchon possède une énergie cinétique suffisante pour transmettre audit matériau pulvérulent un choc qui assure la désagglomération dudit matériau pulvérulent.

Selon une forme de réalisation avantageuse, ladite substance est un matériau solide pulvérulent, ledit réservoir de substance comporte une partie convergente qui converge vers l'orifice de sortie, ladite partie convergente a une forme, et l'orifice de sortie a une section, adaptées à permettre la rétention du matériau pulvérulent par effet de voûte au voisinage de l'orifice de sortie, pendant la manipulation du dispositif avant son actionnement. Avantageusement, dans ce cas, lorsque lesdits moyens pour limiter le mouvement du bouchon arrêtent le déplacement dudit bouchon, ledit bouchon possède une énergie cinétique suffisante pour transmettre audit matériau pulvérulent un choc qui assure le dévoûtage du métariau pulvérulent, et facilite ainsi sa projection au travers de l'orifice de sortie par le flux de gaz comprimé. Pour assurer un bon effet de voûte, avec les poudres pharmaceutiques usuelles, l'orifice de sortie du réservoir de substance peut avoir un diamètre inférieur ou égal à 1,5 mm, éventuellement inférieur ou égal à 1 mm, éventuellement inférieur ou égal à 0,5 mm.

Selon une forme de réalisation :
- le réservoir de ladite substance est compris dans un embout-poussoir ayant une partie arrière cylindrique creuse qui communique avec l'orifice d'entrée de gaz comprimé du réservoir,
- les moyens de compression de gaz comportent un piston creux, qui est emboîté dans la partie arrière de l'embout-poussoir, et un cylindre doté d'un fond, le cylindre coulissant axialement sur le piston, et
- le piston creux définit le canal intermédiaire.

Avantageusement, le piston creux est réalisé dans un matériau therrnoplastique relativement souple, tel que le polyéthylène.

Selon une forme de réalisation, ledit canal intermédiaire comporte une réserve de liquide, et des moyens de rétention de liquide disposés entre le bouchon et la réserve de gaz. Avantageusement, lesdits moyens de rétention sont un canal capillaire.

Selon une autre forme de réalisation, la substance est un matériau solide pulvérulent, le réservoir de substance comporte une barrière fixe qui maintient la dose de matériau pulvérulent dans une position éloignée de l'orifice d'entrée de gaz comprimé, le réservoir de ladite substance est amovible, ledits moyens de compression de gaz constituent une pompe à air dotée d'un clapet d'entrée et d'un moyen élastique de rappel, ledit tronçon de section réduite du canal intermédiaire comporte une extrémité qui est évasée vers l'orifice d'entrée du réservoir, le bouchon est rappelé par un moyen élastique de rappel du bouchon, vers une position dans laquelle il obture ladite extrémité évasée dudit tronçon de section réduite, et ladite extrémité évasée présente une conicité suffisamment faible pour permettre le coincement du bouchon dans ladite extrémité évasée sous la seule action du moyen élastique de rappel du bouchon. Ladite conicité peut avantageusement être inférieure ou égale a 10°. Ladite barrière fixe peut constituer lesdits moyens pour limiter le mouvement du bouchon et lesdits moyens pour garantir le passage du flux de gaz comprimé peuvent comporter au moins une ouverture qui traverse ladite barrière fixe.

D'autes caractéristiques et avantages de l'invention apparaîtront au cours de la description suivante de plusieurs formes de réalisation de l'invention, donnée à titre d'exemple non limitatif, en regard des dessins joints.

Sur les dessins :
- la figure 1 est une vue en coupe d'une première forme de réalisation du dispositif selon l'invention,
- la figure 2 est une vue analogue à la figure 1 pour une autre forme de réalisation du dispositif de l'invention,
- la figure 3 est une vue analogue à la figure 1 pour une autre forme de réalisation du dispositif de l'invention,
- la figure 4 est une vue analogue à la figure 1 pour une autre forme de réalisation du dispositif selon l'invention,
- la figure 5 est une vue de détail agrandie de la figure 4,
- la figure 6 est une vue en coupe selon la ligne VI-VI de la figure 5, et
- la figure 7 est une vue en coupe d'une autre forme de réalisation du dispositif selon l'invention.

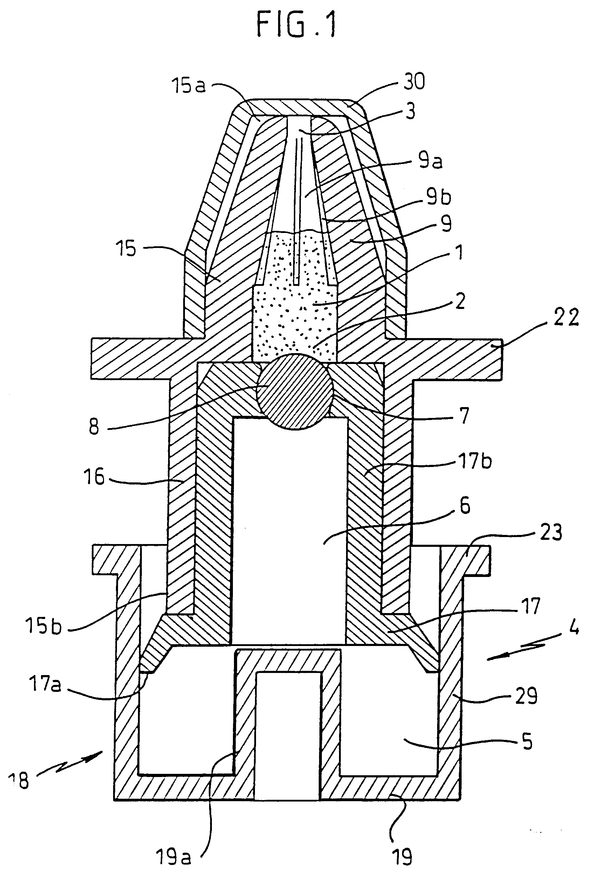

En référence à la figure 1, selon une première forme de réalisation de l'invention, le dispositif comporte un embout-poussoir 15 qui délimite un réservoir 1 contenant une substance à projeter, par exemple un matériau solide pulvérulent. Ce matériau solide pulvérulent sera généralement un médicament à usage nasal. L'embout 15 s'étend axialement entre une extremité avant 15a et une extrémité arrière 15b. A son extrémité avant 15a, l'embout 15 comporte un orifice de sortie 3, qui communique avec le réservoir 1 de substance. L'embout 15 présente une couronne radiale extérieure 22, qui sert de point d'appui pour les doigts d'un utilisateur. L'embout-poussoir 15 comporte une paroi tronconique 9, qui converge vers l'orifice de sortie 3 et qui s'étend entre l'extrémité avant 15a et la couronne radiale extérieure 22. En outre, l'embout-poussoir 15 comporte une paroi cylindrique 16, qui s'étend axialement entre la couronne 22 et l'extrémité arrière 15b. En outre, la paroi tronconique 9 comporte une surface intérieure dans laquelle sont formées des nervures axiales 9b, dont l'utilité sera vue plus loin. L'embout-poussoir 15 qui vient d'être décrit peut être moulé en matériau thermoplastique.

Lorsque le dispositif selon l'invention est stocké avant son utilisation, l'extrémité avant 15a de l'embout-poussoir 15 est recourverte par un capuchon 30, qui peut être formé en matière plastique ou en tout autre matériau, et fixé à l'embout-poussoir 15 par emboîtement serré, encliquetagae, vissage, ou tout autre moyen.

Le dispositif selon l'invention comporte en outre un piston 17, qui est emboîté dans la paroi cylindrique 16 de l'embout-poussoir. Le piston 17 comporte une lèvre périphérique d'étanchéité 17a, qui saille radialement à l'extérieur de la paroi cylindrique 16 au voisinage de l'extrémité arrière 15b de l'embout-poussoir, et une tige de fixation 17b qui est emboîtée dans la paroi cylindrique 16 de l'embout-poussoir 15. La tige de fixation 17b présente un canal central axial 6 qui la traverse, et qui communique avec la réservoir 1 de substance. Le canal axial présente un tronçon 7 de section rétrécie, qui définit un orifice d'entrée 2 du réservoir 1. Dans le tronçon 7 est emboîtée à force une bille ou un bouchon 8 d'une autre forme, de façon à obturer l'orifice d'entrée 2 du réservoir 1 de substance. Le piston 17 est avantageusement moulé dans un matériau thermoplastique relativement souple, par exemple le polyéthylène. La bille peut être réalisée, par exemple, en acier inoxydable ou encore en polyéthylène haute densité.

Le dispositif selon l'invention comporte en outre un cylindre 18 qui peut être moulé en matière plastique. Le cylindre 18 présente un fond 19 et une paroi latérale cylindrique 29 qui s'étend axialement entre le fond 19 et une extrémité ouverte 23. La lèvre périphérique d'étanchéité 17a du piston 17 coulisse avec étanchéité à l'intérieur de la paroi latérale cylindrique 22. Le piston 17 et le cylindre 18 définissent ainsi un système de compression 4 qui contient une réserve de gaz 5, qui peut être remplie d'air ou d'un autre gaz. Avantageusement, le fond 19 du cylindre 18 peut comporter une partie centrale 19a qui saille vers le canal central 6 du piston, et qui est adapté à pénétrer dans ledit canal central 6, lors de l'actionnement du dipositif, de façon à augmenter la pression régnant dans la réserve de gaz 5 lors de l'actionnenemt.

Lorsqu'on veut utiliser le dispositif selon l'invention, on enlève le capuchon 30, on insère l'extrémité avant 15a de l'embout-poussoir à l'intérieur d'une narine, puis on appuie sur le fond 19 du cylindre 18 en maintenant la couronne 22 à l'aide de deux doigts, ce qui a pour effet d'augmenter la pression du gaz dans la réserve 5. Lorsque la pression du gaz a atteint une valeur prédéterminée, qui dépend du serrage de la bille 8 dans le tronçon rétréci 7, la bille est éjectée vers le réservoir 1, de sorte que le gaz comprimé dans la réserve 5 peut s'échapper en entraînant avec lui la poudre contenue dans le réservoir 1. La bille 8 ne risque pas d'obturer l'orifice de sortie 3, grâce aux nervures 9b situées à l'intérieur de la paroi tronconique 9.

Cette forme de réalisation est particulièrement avantageuse, dans la mesure où le piston 17 peut être réalisé dans un matériau souple, ce qui permet une bonne étanchéité du piston 17 avec le cylindre 18 et avec la bille 8, et ce qui permet aussi un bon serrage élastique de la bille 8 à l'intérieur du tronçon rétréci 7.

Avantageusement, la forme de la paroi tronconique 9 et le diamètre de l'orifice de sortie 3 sont tels que, lorsque le capuchon est enlevé, pendant la manipulation du dispositif avant son actionnement, la dose de poudre contenue dans le réservoir 1 soit retenue par effet de voûte à l'intérieur du réservoir 1, par un phénomène similaire à celui qui survient parfois accidentellement dans les silos de stockage de matériaux pulvérulents. De cette façon, même lorsque l'orifice 3 est dirigé vers le bas après enlèvement du capuchon, la dose de poudre contenue dans le réservoir 1 ne s'échappe pas par l'orifice 3 avant l'actionnement du dispositif. L'orifice 3 peut avoir un diamètre inférieur ou égal à 1,5 mm, avantageusement voisin de 1 mm, éventuellement inférieur à 1 mm ou 0,5 mm, avec les poudres pharmaceutiques classique destinées à la pulvérisation nasale.

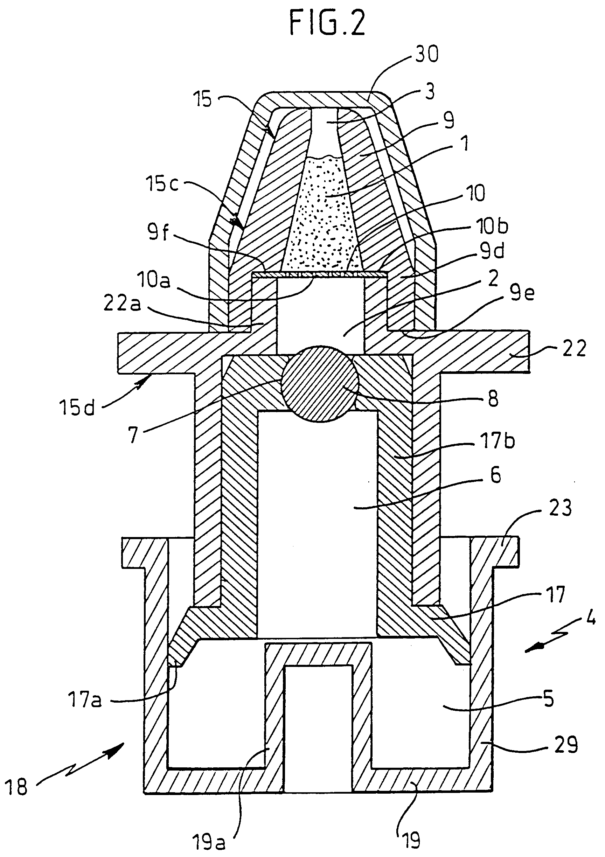

La figure 2 représente une variante du dispositif de la figure 1. La structure générale du dispositif de la figure 2 est similaire à la structure du dispositif de la figure 1, de sorte que le dispositif de la figure 2 ne sera pas décrit de nouveau en détail. Le dispositif de la figure 2 se différencie du dispositif de la figure 1 en ce que la paroi tronconique 9 ne l'embout-poussoir 15 ne comporte pas de nervure intérieure 9b. D'autre part, l'embout-poussoir 15 est ici formé en deux parties, une partie avant 15c comportant la paroi tronconique 9 et une paroi arrière 15d comportant la couronne 22 et la paroi cylindrique 16. La paroi tronconique 9 se prolonge axialement vers la couronne 22 par une paroi cylindrique 9d de faible longueur qui s'étend jusqu'à une extrémité axiale 9e, en formant un épaulement intérieur 9f. En outre, la couronne 22 comporte une paroi cylindrique 22a qui est adaptée à s'emboîter dans la paroi cylindrique 9d. La paroi cylindrique 9d peut être fixée à l'intérieur de la paroi cylindrique 22a par emboîtement à force, par encliquetage, par collage ou par tout autre moyen.

Le dispositif comporte en outre en une grille 10 à maille fine qui présente une périphérie 10b fixée par coincement entre l'épaulement 9f et la paroi cylindirque 22a. La grille 10 peut être constituée par un entrecroissement de fils métalliques solidarisés ou bien elle peut être réalisée par une plaque métallique ou en matière plastique perçée de trous 10a. Les trous 10a ont une taille suffisamment faible pour ne pas être traversés par la poudre contenue dans le réservoir 1. La dose de poudre est ainsi délimitée par la paroi tronconique 9 et la grille 10, et elle est maintenue éloignée de la bille 8.

Lors de l'actionnement du dispositif, la bille 8 vient heurter brutalement la grille 10. L'énergie cynétique accumulée par la bille 10 est suffisante pour creér un choc qui décolle la dose de poudre par rapport à la grille 10, ce qui facilite le passage du gaz au travers de la grille 10. En outre, le choc a pour effet de désagglomérer la poudre contenue dans le réservoir 1, ce qui facilite l'entraînement ultérieur par le flux de gaz comprimé. En outre, le choc entraîne "le dévoûtage" de la dose de poudre autour de l'orifice de sortir 3, c'est-à-dire la suppression de l'effet de voûte qui empêchait la sortie de la poudre par l'orifice 3.

Le fait que la dose de poudre soit maintenue écartée de la bille 8 par la grille 10 permet en outre d'assurer une répartition uniforme du flux de gaz comprimé dans toute la section du réservoir 1, de façon que toute la dose soit entraînée dans le flux de gaz comprimé : on évite ainsi les volumes morts non-balayés par le flux de gaz comprimé.

Avantageusement, la grille 10 présente une certaine flexiblité, de sorte que le choc de la bille sur la grille entraîne une déformation qui a tendance à ouvrir les mailles ou les pores de la grille du côté de la poudre, ce qui favorise le passage d'air.

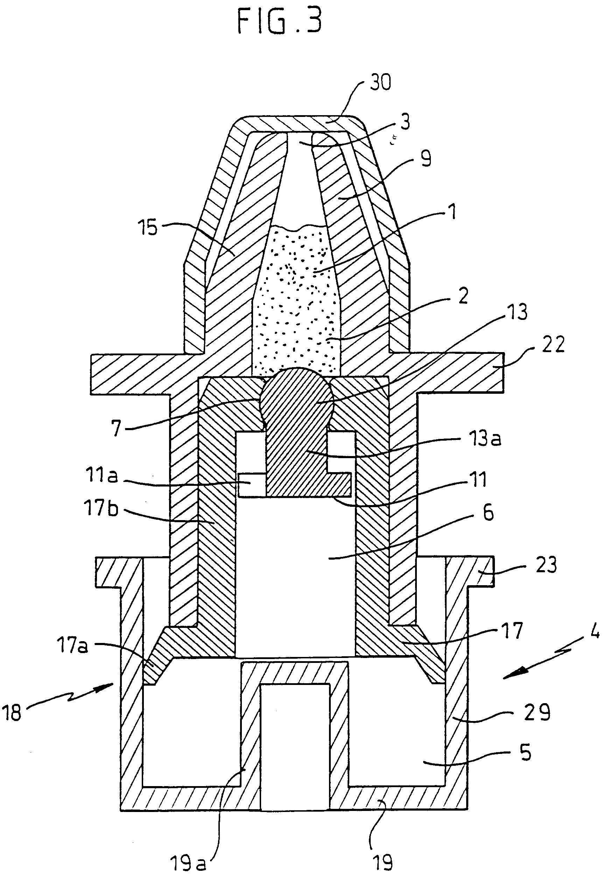

La figure 3 représente une autre variante du dispositif de la figure 1 qui présente une structure voisine, et qui ne sera pas décrite à nouveau en détail.

A la différence du dispositif de la figure 1, le dispositif comporte non plus une bille, mais un bouchon 13 qui se prolonge vers la réserve de gaz 5 par une tige 13a dotée d'un talon 11 saillant radialement vers l'extérieur. Le talon 11 comporte des passages 11a pour le flux de gaz comprimé. Lors de l'actionnement, le bouchon 13 est éjecté vers le réservoir de substance 1, mais son mouvement est limité par le talon 11 qui bute contre le tronçon 7 de section rétrécie du canal intermédiaire 6.

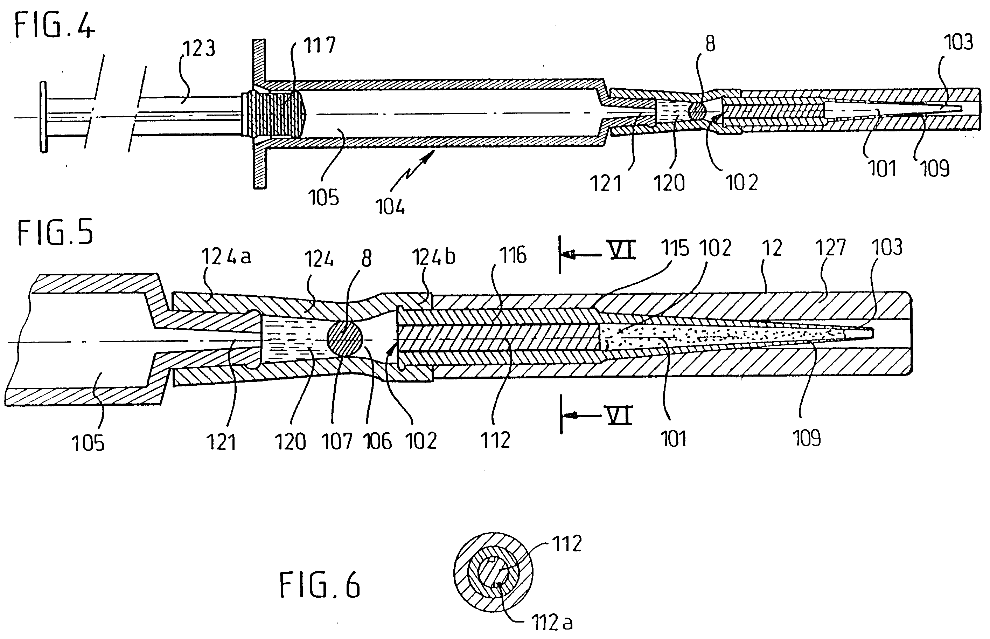

Les figures 4 à 6 représentent une autre forme de réalisation du dispositif selon l'invention. Comme représenté sur la figure 4, le dispositif comporte une seringue 104, dotée d'une réserve de gaz 105 cylindrique dans laquelle coulisse un piston 117 muni d'une tige d'actionnement 123. Le réservoir 105 et la tige d'actionnement 123 peuvent être moulés en matière plastique ; le piston est avantageusement réalisé en élastomère. La seringue 104 présente un canal de sortie capillaire 121, qui est formé dans une embout d'extrémité 122.

Le dispositif comporte en outre un tronçon de tube 124 qui s'étend axialement entre une première extrémité 124a et une deuxième extrémité 124b. La première extrémité 124a du tronçon de tube 124 est emboîtée à force sur l'embout d'extrémité 122 de la seringue 104. Le tronçon de tube 124 définit un canal intermédiaire 106, présentant un tronçon 107 de section réduite. Une bille est coincée dans le tronçon 107 de section réduite, en obstruant ledit tronçon 107.

Le dispositif comporte en outre un embout 115 qui présente une paroi latérale cylindrique 116, emboîtée à force dans la deuxième extrémité 124b du tronçon de tube 124, ladite paroi cylindrique 116 se prolongeant vers l'avant par une paroi tronconique 109, jusqu'à un orifice de sortie 103. Une tige 112, comportant deux rainures latérales axiales 112a, est emboîtée à force à l'intérieur de la paroi latérale cylindrique 116.

En outre, dans cet exemple particulier, un manchon 127 de forme extérieure cylindrique est emboîté sur la paroi latérale 116 et la paroi tronconique 109 afin d'éviter qu'un utilisateur ne puisse se blesser avec l'extrémité pointue de la paroi tronconique 109.

Le manchon 127 est coiffé d'un capuchon (non représenté) pendant le stockage.

La paroi tronconique 109 et la tige 126 délimitent une réserve de poudre 101 qui présente un orifice d'entrée 102. La rainure 126a a une dimension suffisamment faible pour que la poudre contenue dans le réservoir 101 n'entre pas dans ladite rainure 126a, et l'orifice de sortie 103 a une dimension suffisamment faible pour que la poudre ne sorte par l'orifice pendant la manipulation du dispositif avant son actionnement, en créant un effet de voûte, comme il a été vu précédemment. En outre, le tronçon de tube 124, la bille 8 et le canal capillaire 121 délimitent un réserve de liquide 120, qui est expulsé en même temps que la poudre lors de l'actionnement. Le canal capillaire 121 a un diamètre suffisamment petit pour éviter que le liquide de la réserve 120 ne pénètre dans la réserve de gaz 105.

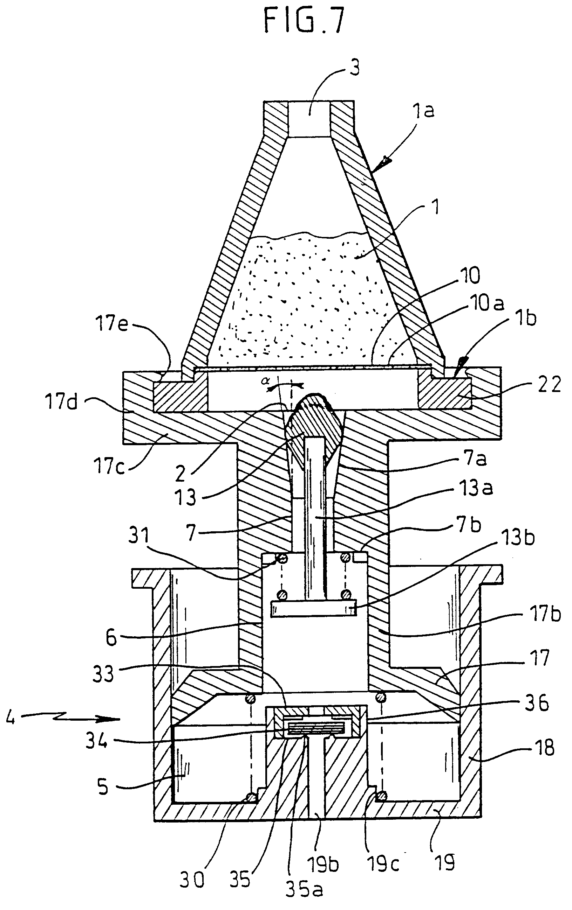

Le dispositif représenté sur la figure 7 est une variante de la figure 2 qui est conçue pour être rechargeable. Le dispositif de la figure 7 comporte un réservoir 1 amovible, qui est formé en deux pièces. Le réservoir 1 comporte une partie avant 1a et une partie arrière 1b, qui sont assemblées par emboîtement à force, par collage, par encliquetage ou par tout autre moyen, en emprisonnant entre elles une grille 10, similaire à celle décrite en regard de la figure 2. La partie avant 1a du réservoir 1 présente une forme générale tronconique, et présente un orifice de sortie 3. Le réservoir 1 est conçu pour contenir une substance sous forme pulvérulente, la grille est conçue pour retenir ladite substance pulvérulente à l'intérieur de la partie avant 1a, et la forme de la partie avant 1a ainsi que la section de l'orifice de sortie 3 sont prévues pour empêcher la sortie accidentelle de la poudre pendant la manipulation du dispositif.

La partie arrière 1b du réservoir 1 comporte une couronne radiale extérieure 22.

Le dispositif comporte en outre une pompe à air 4, comportant un cylindre 18 et un piston 17 coulissant dans le cylindre. Le piston et le cylindre définissent une chambre de pompe 5, et un ressort de rappel 30 est interposé entre eux. Le cylindre 18 comporte un fond 19, doté d'une ouverture d'entrée d'air 19b qui communique avec un clapet d'entrée. Dans l'exemple représenté, l'orifice d'entrée 19b débouche à l'intérieur du cylindre 18, au centre d'une surface 35 sensiblement radiale. Sur la surface 35, un cordon d'étanchéité 35a est formé autour du débouché de l'orifice d'entrée 19b. La surface radiale 35 est entourée par une paroi cylindrique axiale 36, qui reçoit un porte-clapet 33. Un joint plat 34, en élastomère, est emprisonné entre le porte-clapet 33 et la surface radiale 35, et ledit joint 34 est adapté à s'appliquer de façon étanche sur le cordon d'étanchéité 35a lorsqu'une surpression règne à l'intérieur du cylindre 18. Des exemples de tels clapets d'entrée sont divulgués dans le document EP-A-0 377 536, et dans le brevet américain correspondant US-A-4 966 535. Le fond 19 présente des reliefs 19c de centrage du ressort 30.

Le piston 17 présente une tige creuse 17b, qui définit un canal intermédiaire entre la pompe à air 4 et le réservoir 1. La tige 17b s'étend axialement vers le réservoir 1 jusqu'à une couronne 17c, qui s'étend radialement vers l'extérieur. La couronne 17c est prolongée axialement vers le réservoir 1 par une paroi latérale cylindrique 17b, qui s'étend jusqu'à des moyens d'encliquetage 17e. Les moyens d'encliquetage 17 peuvent être par exemple, une nervure intérieure. La couronne 22 du réservoir 1 est reçue à l'intérieur de la paroi latérale cylindrique 17d, et elle est maintenue au contact de la couronne 17c par les moyens d'encliquetage 17e. La couronne 22 du réservoir 12 est en contact étanche avec la paroi latérale cylindrique 17d ou la couronne 17c. Avantageusement, le piston 17 est formé dans un matériau relativement souple, tel que du polyéthylène, de sorte que l'étanchéité entre la couronne 22 et la paroi latérale cylindrique 17 peut être obtenue par serrage élastique de la couronne 22 à l'intérieur de la paroi cylindrique 17d.

Comme dans les exemples précédents, le canal intermédiaire 6 présente un tronçon 7 de section rétrécie au voisinage du réservoir 1. Le tronçon 7 définit un orifice d'entrée 2 du réservoir 1. Dans l'exemple représenté, le tronçon 7 de section rétrécie présente au voisinage du réservoir 1, une extrémité élargie 7b évasée vers le réservoir 1. L'extrémité 7b a aussi une forme conique, avec un angle de conicité α de préférence inférieur ou égal à 10°. En outre, le tronçon 7 de section rétrécie définit aussi un épaulement 7a, dirigé vers le fond 19 du cylindre 18.

Le dispositif comporte en outre un bouchon 13, qui est coincé dans l'extrémité évasée 7b du tronçon 7. Une tige 13a est emboîtée à force dans ledit bouchon 13, et ladite tige s'étend axialement vers le fond 19 du cylindre 19, jusqu'à un collet 13b, disposé à l'intérieur de la partie la plus large du canal intermédiaire 6. Un ressort de rappel 31 du bouchon est disposé entre l'épaulement 7a et le collet 13b. Le ressort de rappel 31 est généralement de faible raideur, de façon à ne pas gêner le déplacement du bouchon 13 vers le réservoir 1 lors de l'actionnement. En outre, la tige 13a possède une longueur suffisante pour permettre au bouchon 13 de venir frapper la grille 10 lors de l'actionnement. Enfin, grâce à la faible conicité de l'extrémité évasée 7b du canal intermédiaire, le bouchon 13 vient se coincer dans ladite extrémité évasée 7b après chaque actionnement, sous la seule action du ressort de rappel 31 du bouchon, et ce malgré la faible raideur dudit ressort 31.

## Revendications

1. Dispositif à gaz comprimé pour projeter sous forme finement divisée une dose unique d'une substance fluide, comportant:
- un réservoir (1, 101) contenant ladite dose unique, ledit réservoir ayant un orifice d'entrée de gaz comprimé (2, 102) et un orifice de sortie (3, 103),
- des moyens de compression de gaz (4, 104) à actionnement manuel, lesdits moyens de compression de gaz (4, 104) comportant une réserve de gaz (5, 105) qui communique avec l'orifice d'entrée (2, 102) du réservoir (1, 101) par l'intermédiaire d'un canal intermédiaire (6, 16) comportant un tronçon de section rétrécie (7, 107), ledit tronçon de section rétrécie étant fermé de façon étanche par un bouchon (8, 103) qui est coincé dans ledit tronçon de section rétrécie, de façon que lorsque la pression du gaz contenu dans la réserve de gaz (5, 105) atteint un seuil prédéterminé, ledit bouchon (8, 103) soit expulsé dudit tronçon de section rétrécie (7, 107) vers le réservoir (1, 101) de substance, en libérant ledit canal intermédiaire (6, 106),
le dispositif comportant des moyens (9, 10, 11, 112) pour limiter le déplacement du bouchon (8, 13) vers l'orifice de sortie du réservoir de substance, en définissant ainsi une position de butée du bouchon,
caractérisé en ce que lesdits moyens (9, 10, 11, 112) pour limiter le déplacement du bouchon présentent des moyens (9b, 10a, 11a, 112a) pour garantir le passage du flux de gaz comprimé lorsque le bouchon est dans sa position de butée.

2. Dispositif selon la revendication 1, dans lequel le réservoir de substance (1) comporte une partie convergente (9), qui converge vers l'orifice de sortie (3), ladite partie convergente (9) constitue lesdits moyens pour limiter le mouvement du bouchon, et ladite partie convergente (9) comporte des reliefs intérieurs (9c) qui constituent lesdits moyens (9c) pour garantir le passage du flux de gaz comprimé.

3. Dispositif selon la revendication 1, dans lequel la substance est un matériau solide pulvérulent le réservoir de substance (1) comporte une barrière fixe (10, 112) qui maintient la dose de matériau pulvérulent dans une position éloignée de l'orifice d'entrée de gaz comprimé (2), ladite barrière fixe constitue lesdits moyens pour limiter le mouvement du bouchon, et lesdits moyens pour garantir le passage du flux de gaz comprimé comportent au moins une ouverture (10a, 112a) qui traverse ladite barrière fixe.

4. Dispositif selon la revendication 3, dans lequel lorsque le bouchon (8) entre en contact avec ladite barrière fixe (10, 112), ledit bouchon possède une énergie cinétique suffisante pour que le choc du bouchon sur ladite barrière fixe entraîne un décollement de la dose de matériau pulvérulent par rapport à ladite barrière fixe.

5. Dispositif selon la revendication 3 ou 4, dans lequel ladite barrière fixe est un élément en forme de membrane (10).

6. Dispositif selon la revendication 5, dans lequel la membrane (10) possède une certaine flexibilité.

7. Dispositif selon la revendication 3 ou 4, dans lequel ladite barrière fixe comporte une tige centrale pleine (112) dotée d'au moins une rainure latérale (112a) axiale constituant la au moins une ouverture de passage du flux de gaz comprimé.

8. Dispositif selon la revendication 1, dans lequel le bouchon (13) comporte un talon élargi (11) qui coopère avec le tronçon de section rétrécie (7) du canal intermédiaire (6) pour limiter le mouvement du bouchon vers le réservoir (1) de substance, et ledit talon comporte au moins une ouverture (11a) qui constitue lesdits moyens pour garantir le passage du flux de gaz comprimé.

9. Dispositif selon la revendication 1, dans lequel ladite substance est un matériau solide pulvérulent, et lorsque lesdits moyens (9, 10, 11, 112) pour limiter le mouvement du bouchon arrêtent le déplacement dudit bouchon, ledit bouchon possède une énergie cinétique suffisante pour transmettre audit matériau pulvérulent un choc qui assure la désagglomération dudit matériau pulvérulent.

10. Dispositif selon la revendication 1, dans lequel ladite substance est un matériau solide pulvérulent, ledit réservoir de substance (1, 101) comporte une partie convergente (9, 109) qui converge vers l'orifice de sortie (3, 103), ladite partie convergente a une forme, et l'orifice de sortie a une section, adaptées à permettre la rétention du matériau pulvérulent par effet de voûte au voisinage de l'orifice de sortie, pendant la manipulation du dispositif avant son actionnement.

11. Dispositif selon la revendication 10, dans lequel lorsque lesdits moyens (9, 10, 11, 112) pour limiter le mouvement du bouchon arrêtent le déplacement dudit bouchon, ledit bouchon possède une énergie cinétique suffisante pour transmettre audit matériau pulvérulent un choc qui assure le dévoûtage du métariau pulvérulent, et facilite ainsi sa projection au travers de l'orifice de sortie (3, 103) par le flux de gaz comprimé.

12. Dispositif selon la revendication 10, dans lequel l'orifice de sortie (3, 103) a un diamètre inférieur ou égal à 1,5 mm.

13. Dispositif selon la revendication 10, dans lequel l'orifice de sortie (3, 103) a un diamètre inférieur ou égal à 1 mm.

14. Dispositif selon la revendication 10, dans lequel l'orifice de sortie (3, 103) a un diamètre inférieur ou égal à 0,5 mm.

15. Dispositif selon la revendication 1, dans lequel:
- le réservoir (3) de ladite substance est compris dans un embout-poussoir (15) ayant une partie arrière cylindrique creuse (16) qui communique avec l'orifice d'entrée (2) de gaz comprimé du réservoir (3),
- les moyens de compression de gaz (4) comportent un piston (17) creux, qui est emboîté dans la partie arrière (16) de l'embout-poussoir (15), et un cylindre (18) doté d'un fond (19), le cylindre (18) coulissant axialement sur le piston (17), et
- le piston creux (17) définit le canal intermédiaire (6).

16. Dispositif selon la revendication 15, dans lequel le piston creux (17) est réalisé dans un matériau thermoplastique souple.

17. Dispositif selon la revendication 16, dans lequel ledit matériau thermoplastique souple est le polyéthylène.

18. Dispositif selon la revendication 1, dans lequel ledit canal intermédiaire (106) comporte une réserve de liquide (120), et des moyens de rétention de liquide (121) disposés entre le bouchon et la réserve de gaz (4).

19. Dispositif selon la revendication 18, dans lequel lesdits moyens de rétention sont un canal capillaire (121).

20. Dispositif selon la revendication 1, dans lequel la substance est un matériau solide pulvérulent, le réservoir de substance (1) comporte une barrière fixe (10) qui maintient la dose de matériau pulvérulent dans une position éloignée de l'orifice d'entrée de gaz comprimé (2), le réservoir (1) de ladite substance est amovible, ledits moyens de compression de gaz (4) constituent une pompe à air dotée d'un clapet d'entrée (33, 34) et d'un moyen élastique de rappel (30), ledit tronçon de section réduite (7) du canal intermédiaire (6) comporte une extrémité (7b) qui est évasée vers l'orifice d'entrée (2) du réservoir, le bouchon (13) est rappelé par un moyen élastique (31) de rappel du bouchon, vers une position dans laquelle il obture ladite extrémité évasée (7b) dudit tronçon de section réduite (7), et ladite extrémité évasée (7b) présente une conicité suffisamment faible pour permettre le coincement du bouchon (13) dans ladite extrémité évasée sous la seule action du moyen élastique (31) de rappel du bouchon.

21. Dispositif selon la revendication 20, dans lequel ladite conicité est inférieure ou égale à 10°.

22. Dispositif selon la revendication 20 ou la revendication 21, dans lequel ladite barrière fixe (10) constitue lesdits moyens pour limiter le mouvement du bouchon, et lesdits moyens pour garantir le passage du flux de gaz comprimé comporte au moins une ouverture (10a) qui traverse ladite barrière fixe.

## Claims

1. A compressed gas device for spraying a single dose of a fluid substance in finely divided form, the device comprising:
a tank (1, 101) containing said single dose, said tank having an inlet orifice (2, 102) for compressed gas and an outlet orifice (3, 103);
manually-actuated gas compression means (4, 104), said gas compression means (4, 104) comprising a supply of gas (5, 105) communicating with the inlet orifice (2, 102) of the tank (1, 101) via an intermediate channel (6, 16) including a length of narrowed section (7, 107), said length of narrowed section being closed in sealed manner by a plug (8, 103) which is held in said length of narrowed section so that when the pressure of the gas contained in the supply of gas (5, 105) reaches a predetermined threshold, said plug (8, 103) is expelled from said length of narrowed section (7, 107) towards the tank (1, 101) of substance, thereby opening said intermediate channel (6, 106);
the device including means (9, 10, 11, 112) for limiting the displacement of the plug (8, 13) towards the outlet orifice of the tank of substance, thereby defining an abutment position for the plug;
the device being characterized in that said means (9, 10, 11, 112) for limiting displacement of the plug include means (9c, 10a, 11a, 112a) for guaranteeing that the compressed gas can continue to flow when the plug is in its abutment position.

2. A device according to claim 1, wherein the tank of substance (1) includes a convergent portion (9) which converges towards the outlet orifice (3), said convergent portion (9) constituting said means for limiting the movement of the plug, and said convergent portion (9) including inside relief (9c) constituting said means (9c) for guaranteeing that the compressed gas can flow.

3. A device according to claim 1, wherein the substance is a solid powder material, the tank of substance (1) includes a fixed barrier (10, 112) which holds the dose of powder material in a position that is distant from the inlet orifice (2) for compressed gas, said fixed barrier constituting said means for limiting the movement of the plug, and said means for guaranteeing that the compressed gas can flow include at least one opening (10a, 112a) through said fixed barrier.

4. A device according to claim 3, wherein when the plug (8) comes into contact with said fixed barrier (10, 112), and said plug has sufficient kinetic energy for the shock of the plug against the fixed barrier to cause the dose of powder material to be unstuck from said fixed barrier.

5. A device according to claim 3 or 4, wherein said fixed barrier is an element in the form of a diaphragm (10).

6. A device according to claim 5, wherein the diaphragm (10) has a degree of flexibility.

7. A device according to claim 3 or 4, wherein said fixed barrier includes a solid central rod (112) provided with at least one axial lateral groove (112a) constituting the at least one opening for allowing the compressed gas to flow.

8. A device according to claim 1, wherein the plug (13) includes an enlarged catch (11) which co-operates with the length of narrowed section (7) of the intermediate channel (6) to limit the movement of the plug towards the tank (1) of substance, said catch including at least one opening (11a) which constitutes said means for guaranteeing that the compressed gas can flow.

9. A device according to claim 1, wherein said substance is a solid powder material, and when said means (9, 10, 11, 112) for limiting the movement of the plug stop displacement of said plug, said plug possesses sufficient kinetic energy to transmit a shock to said powder material that ensures that said powder material breaks up.

10. A device according to claim 1, wherein said substance is a solid powder material, said tank of substance (1, 101) includes a convergent portion (9, 109) that converges towards the outlet orifice (3, 103), said convergent portion being of a shape and the outlet orifice being of a section that are adapted to enable the powder material to be retained by a bridging effect in the vicinity of the outlet orifice while the device is being handled prior to being actuated.

11. A device according to claim 10, wherein when said means (9, 10, 11, 112) for limiting the movement of the plug stop the displacement of the plug, said plug possesses sufficient kinetic energy to transmit a shock to said powder material that breaks the bridging of the powder material and thus facilities spraying of the powder material through the outlet orifice (3, 103) by the flow of compressed gas.

12. A device according to claim 10, wherein the outlet orifice (3, 103) has a diameter that is less than or equal to 1.5 mm.

13. A device according to claim 10, wherein the outlet orifice (3, 103) has a diameter that is less than or equal to 1 mm.

14. A device according to claim 10, wherein the outlet orifice (3, 103) has a diameter that is less than or equal to 0.5 mm.

15. A device according to claim 1, wherein:
the tank (3) of said substance is included in a pusher-endpiece (15) having a hollow cylindrical rear portion (16) in communication with the compressed gas inlet orifice (2) of the tank (3);
the gas compression means (4) include a hollow piston (17) which is engaged in the rear portion (16) of the pusher-endpiece (15), and a cylinder (18) provided with an end wall (19), the cylinder (18) sliding axially over the piston (16); and
the hollow piston (17) defines the intermediate channel (6).

16. A device according to claim 15, wherein the hollow piston (17) is made of a flexible thermoplastic material.

17. A device according to claim 16, wherein the said flexible thermoplastic material is polyethylene.

18. A device according to claim 1, wherein the said intermediate channel (106) includes a supply of liquid (120) and liquid-retaining means (121) are disposed between the plug and the supply of gas (4).

19. A device according to claim 18, wherein the liquid-retaining means are constituted by a capillary channel (121).

20. A device according to claim 1, wherein the substance is a solid powder material, the tank of substance (1) includes a fixed barrier (10) which holds the dose of powder material in a position distant from the inlet orifice for compressed gas (2), the tank (1) of said substance is removable, said gas compression means (4) constitute an air pump provided with an inlet check valve (33, 34) and with resilient return means (30), said length of narrowed section (7) of the intermediate channel (6) includes an end (7b) which flares towards the inlet orifice (2) of the tank, the plug (13) is urged by resilient return means (31) for the plug towards a position in which it closes said flared end (7b) of the length of narrowed section (7), and said flared end (7b) has a cone angle that is small enough to enable the plug (13) to be jammed in said flared end solely under drive from the resilient plug return means (31).

21. A device according to claim 20, wherein the half-angle at the apex of the said flared end is less than or equal to 10°.

22. A device according to claim 20 or claim 21, wherein said fixed barrier (10) constitutes said means for limiting the movement of the plug, and said means for guaranteeing that compressed gas can flow include at least one opening (10a) passing through said fixed barrier.

## Patentansprüche

1. Druckgasvorrichtung zum Versprühen einer Einzeldosis eines Fluids in fein verteilter Form, die folgende Bestandteile umfaßt:
- einen Behälter (1, 101), der die Einzeldosis enthält und eine Eintrittsöffnung (2, 102) für das Druckgas sowie eine Ausgangsöffnung (3, 103) aufweist,
- von Hand betätigbare Einrichtungen (4, 104) zum Komprimieren des Gases, wobei diese Einrichtungen (4, 104) zum Komprimieren des Gases einen Gasvorratsraum (5, 105) umfassen, der mit der Eintrittsöffnung (2, 102) des Behälters (1, 101) vermittels eines Verbindungskanals (6, 16) in Verbindung steht, der einen Abschnitt (7, 107) mit verengtem Querschnitt aufweist, wobei dieser Abschnitt mit verengtem Querschnitt durch einen Pfropfen (8, 103) dicht verschlossen ist, der in den Abschnitt mit verengtem Querschnitt in der Weise eingeklemmt ist, daß dann, wenn der Druck des im Gasvorratsraum (5, 105) enthaltenen Gases einen vorbestimmten Grenzwert erreicht, der Pfropfen (8, 103) aus dem Abschnitt (7, 107) mit verengtem Querschnitt zum Behälter (1, 101) der Substanz hin herausgedrückt wird und dabei den Verbindungskanal (6, 106) freigibt, und
wobei die Vorrichtung Mittel (9, 10, 11, 112) zum Begrenzen der Verschiebung des Pfropfens (8, 13) in Richtung der Ausgangsöffnung des Substanzbehälters umfaßt, die somit eine Anschlagsposition des Pfropfens definieren, dadurch **gekennzeichnet,** daß die Mittel (9, 10, 11, 112) zum Begrenzen der Verschiebung des Pfropfens Mittel (9b, 10a, 11a, 112a) umfassen, die das Hindurchtreten des Druckgasstroms sicherstellen, wenn sich der Pfropfen in seiner Anschlagsposition befindet.

2. Vorrichtung nach Anspruch 1, bei der der Substanzbehälter (1) einen konvergierenden Teil (9) umfaßt, der zur Ausgangsöffnung (3) hin konvergiert, wobei der konvergierende Teil (9) die Mittel zum Begrenzen der Bewegung des Pfropfens bildet und der konvergierende Teil (9) innere Erhabenheiten (9c) aufweist, die die Mittel (9c) bilden, die das Hindurchströmen des Druckgasstromes sicherstellen.

3. Vorrichtung nach Anspruch 1, bei der die Substanz ein festes pulverförmiges Material ist und bei der der Substanzbehälter (1) eine feste Barriere (10, 112) umfaßt, die die Dosis des pulverförmigen Materials in einer von der Eintrittsöffnung (2) für das Druckgas entfernten Position hält, wobei die feste Barriere die Mittel zum Begrenzen der Bewegung des Pfropfens bildet und wobei die Mittel, die das Hindurchtreten des Druckgasstromes sicherstellen, wenigstens eine Öffnung (10a, 112a) umfassen, die sich durch die feste Barriere hindurch erstreckt.

4. Vorrichtung nach Anspruch 3, bei der dann, wenn der Pfropfen (8) in Berührung mit der festen Barriere (10, 112) tritt, der Pfropfen eine kinetische Energie besitzt, die ausreicht, daß der Stoß des Pfropfens auf die feste Barriere eine Ablösung der Dosis des pulverförmigen Materials von der festen Barriere zur Folge hat.

5. Vorrichtung nach Anspruch 3 oder 4, bei der die feste Barriere ein Element in Form einer Membran (10) ist.

6. Vorrichtung nach Anspruch 5, bei der die Membran (10) eine gewisse Flexibilität besitzt.

7. Vorrichtung nach Anspruch 3 oder 4, bei der die feste Barriere eine zentrale Vollstange (112) umfaßt, die wenigstens eine seitliche, sich in axialer Richtung erstreckende Rille (112a) aufweist, die wenigstens eine Durchgangsöffnung für den Druckgasstrom bildet.

8. Vorrichtung nach Anspruch 1, bei der der Pfropfen (13) einen vergrößerten Ansatz (11) umfaßt, der mit dem Abschnitt mit verengtem Querschnitt (7) des Verbindungskanals (6) zusammenwirkt, um die Bewegung des Pfropfens zum Substanzbehälter (1) hin zu begrenzen, und wobei dieser Ansatz wenigstens eine Öffnung (11a) umfaßt, die die Mittel zum Sicherstellen des Durchgangs des Druckgasstromes bildet.

9. Vorrichtung nach Anspruch 1, bei der die Substanz ein festes pulverförmiges Material ist und bei der dann, wenn die Mittel (9, 10, 11, 112) zum Begrenzen der Bewegung des Pfropfens die Verschiebung dieses Pfropfens anhalten, der Pfropfen eine kinetische Energie besitzt, die ausreicht, um auf das pulverförmige Material einen Stoß zu übertragen, der eine Desagglomeration des besagten pulverförmigen Materials sicherstellt.

10. Vorrichtung nach Anspruch 1, bei der die Substanz ein festes pulverförmiges Material ist und der Behälter (1, 101) für die Substanz einen konvergierenden Teil (9, 109) umfaßt, der zur Austrittsöffnung (3, 103) hin konvergiert, wobei der konvergierende Teil eine Form und die Austrittsöffnung einen Querschnitt besitzen, die geeignet sind, das Zurückhalten des pulverförmigen Materials durch einen Gewölbeeffekt in der Nachbarschaft der Ausgangsöffnung während der Handhabung der Vorrichtung vor ihrer Betätigung zu ermöglichen.

11. Vorrichtung nach Anspruch 10, bei der dann, wenn die Mittel (9, 10, 11, 112) zur Begrenzung der Bewegung des Pfropfens die Verschiebung dieses Pfropfens anhalten, der Pfropfen eine kinetische Energie besitzt, die ausreichend ist, um auf das pulverförmige Material einen Stoß auszuüben, der die Aufhebung des Gewölbeeffektes des pulverförmigen Materials sicherstellt und somit sein Ausstoßen durch die Ausgangsöffnung (3, 103) durch den Druckgasstrom zu erleichtern.

12. Vorrichtung nach Anspruch 10, bei der die Austrittsöffnung (3, 103) einen Durchmesser von kleiner/gleich 1,5 mm besitzt.

13. Vorrichtung nach Anspruch 10, bei der die Austrittsöffnung (3, 103) einen Durchmesser kleiner/gleich 1 mm besitzt.

14. Vorrichtung nach Anspruch 10, bei der die Austrittsöffnung (3, 103) einen Durchmesser kleiner/gleich 0,5 mm besitzt.

15. Vorrichtung nach Anspruch 1, bei der
- der Behälter (3) für die Substanz in einem Druckansatzstück (15) enthalten ist, das einen hinteren hohlzylindrischen Teil (16) aufweist, der mit der Druckgas-Eintrittsöffnung (2) des Behälters (3) in Verbindung steht,
- die Einrichtungen (4) zum Komprimieren des Gases einen hohlen Kolben (17) umfassen, der in den hinteren Teil (16) des Druckansatzstückes (15) eingesetzt ist, sowie einen mit einem Boden (19) versehenen Zylinder (18), der in axialer Richtung auf dem Kolben (17) gleitet, und bei der
- der hohle Kolben (17) den Verbindungskanal (6) umgrenzt.

16. Vorrichtung nach Anspruch 15, bei der der hohle Kolben (17) aus einem nachgiebigen thermoplastischen Material hergestellt ist.

17. Vorrichtung nach Anspruch 16, bei der das nachgiebige thermoplastische Material Polyäthylen ist.

18. Vorrichtung nach Anspruch 1, bei der der Verbindungskanal (106) einen Flüssigkeitsvorrat (120) und Mittel zum Zurückhalten der Flüssigkeit (121) umfaßt, die zwischen dem Pfropfen und dem Gas-Vorratsraum (4) angeordnet sind.

19. Vorrichtung nach Anspruch 18, bei der die Rückhaltemittel von einem Kapillarkanal (121) gebildet werden.

20. Vorrichtung nach Anspruch 1, bei der die Substanz ein festes pulverförmiges Material ist, der Behälter für die Substanz (1) eine feste Barriere (10) umfaßt, die die Dosis des pulverförmigen Materials in einer von der Druckgas-Eintrittsöffnung (2) entfernten Position hält, der Behälter (1) für die Substanz abnehmbar ist, bei der die Einrichtungen zum Komprimieren des Gases (4) eine Luftpumpe bilden, die mit einem Eintrittsventil (33, 34) und einer elastischen Rückholvorrichtung (30) versehen ist, bei der der Abschnitt (7) mit vermindertem Querschnitt des Verbindungskanals (6) einen Endbereich (7b) umfaßt, der zur Eintrittsöffnung (2) des Behälters hin erweitert ist, bei der der Pfropfen (13) von einer elastischen Pfropfen-Rückholeinrichtung (31) in eine Position vorgespannt ist, in der er den erweiterten Endbereich (7b) des Verbindungskanals (7) verschließt und bei der der erweiterte Endbereich (7b) eine ausreichend schwache Konizität besitzt, um das Einklemmen des Pfropfens (13) in den erweiterten Endbereich allein unter der Wirkung der elastischen Pfropfen-Rückholvorrichtung (31) zu ermöglichen.

21. Vorrichtung nach Anspruch 20, bei der die Konizität weniger oder gleich 10° ist.

22. Vorrichtung nach Anspruch 20 oder 21, bei der die feste Barriere (10) die Mittel zum Begrenzen der Bewegung des Pfropfens bildet und bei der die Einrichtungen, die das Hindurchtreten des Druckgasstromes sicherstellen, wenigstens eine Öffnung (10a) umfassen, die sich durch die feste Barriere hindurch erstreckt.
